(19) **Europäisches Patentamt**

**European Patent Office**

**Office européen des brevets**

(11) Veröffentlichungsnummer : **0 253 996 B1**

# EUROPÄISCHE PATENTSCHRIFT

(12)

(45) Veröffentlichungstag der Patentschrift :
06.02.91 Patentblatt 91/06

(51) Int. Cl.⁵ : **C07H 15/04**

(21) Anmeldenummer : 87107874.7

(22) Anmeldetag : 01.06.87

(54) Verfahren zur Gewinnung von Butyloligoglycosiden.

(30) Priorität : 23.07.86 DE 3624863

(43) Veröffentlichungstag der Anmeldung :
27.01.88 Patentblatt 88/04

(45) Bekanntmachung des Hinweises auf die
Patenterteilung :
06.02.91 Patentblatt 91/06

(84) Benannte Vertragsstaaten :
AT BE DE ES FR GB IT NL

(56) Entgegenhaltungen :
EP-A- 0 102 558
EP-A- 0 132 046
GB-A- 2 131 802
US-A- 3 565 885

(73) Patentinhaber : HÜLS
AKTIENGESELLSCHAFT
Patentabteilung / PB 15 - Postfach 13 20
D-4370 Marl 1 (DE)

(72) Erfinder : Lüders, Harald, Dr.
Langeoogstrasse 81
D-4350 Recklinghausen (DE)

## Beschreibung

Butylglycoside, Butyloligoglycoside und deren Mischungen sind interessante Polyole, die über Kohlenhydrate, einer nachwachsenden Rohstoffbasis, zugänglich sind. Sie erlangen als Zwischenprodukte bei der Herstellung von Polyurethanen und von Tensiden auf Basis langkettiger Alkylglycoside zunehmende Bedeutung.

Butyloligoglycoside werden im allgemeinen durch Erhitzen von Sacchariden, Butanol und einer Säure hergestellt. Bei diesem Verfahren fallen in merklichem Ausmaße Nebenprodukte an, die den Glycosiden eine unansehnliche Farbe und unangenehmen Geruch verleihen. Diese Nebenprodukte treten insbesondere dann auf, wenn man bei der Herstellung von Polysacchariden ausgeht, die unter verschärften Bedingungen umgesetzt werden müssen.

Außerdem fallen auch farblose Nebenprodukte an, die bei der weiteren Verwendung der Glycoside oder bei Reinigungsoperationen zu Verfärbungen führen können. Dies trifft insbesondere bei der säurekatalysierten Transglycosidierung zu Alkyloligoglucosiden mit langkettigen Alkylresten zu.

Es sind verschiedene Verfahren bekannt, nach denen die Farbe der Alkyloligoglycoside verbessert werden kann. So wird in EP-PS 102 558 ein Verfahren beschrieben, bei dem die Farbqualität der Glycoside durch Zugabe von Alkaliboraten in den Reaktionsansatz verbessert wird. Die Borate werden in einer der katalytischen Schwefelsäure mindestens äquivalenten Menge angewendet. Bei diesem Verfahren kann Stärke nicht als Ausgangsmaterial eingesetzt werden, da die Butanolyse von Stärke durch Borsäuren nicht effektiv katalysiert wird.

Nach US-PS 4 483 979 kann man Farbstoffe aus Alkylpolysacchariden mit langkettigen Alkylresten unter wasserfreien Bedingungen mit polaren Lösungsmitteln extrahieren. Bei diesem Verfahren werden Glycoside mit 1 bis 2 Glycoseeinheiten mitextrahiert. Da die technisch interessanten Butyloligoglucoside mit 1 bis 3 Glucoseeinheiten in Aceton und anderen polaren Lösemitteln gut löslich sind, können diese Produkte nach diesem Verfahren nicht selektiv von farbigen Verunreinigungen befreit werden.

In EP-PS 99 183 werden Alkylglycoside aus wasserhaltigen Polysacchariden und Alkoholen hergestellt. Die Reaktion wird in Gegenwart von Cosolvenzien wie Methanol, Ethanol, Ethylenglykol oder Aceton durchgeführt. Das Reaktionsgemisch weist einen hohen Gehalt an unumgesetzter Glucose auf. Das Reaktionsprodukt ist zwar farblich verbessert, erfordert aber wegen der Cosolvenzien zur Reinigung einen erhöhten Destillationsaufwand.

Die Anwendung von Reduktionsmitteln wie hypophosphorige Säure auf das Reaktionsgemisch Glucose/Butanol/Säure beschreibt EP-PS 77 167. Dieses Verfahren führt bei Monosacchariden zu farbverbesserten Glycosiden, ergibt jedoch beim Einsatz von Stärke unter verschärften Reaktionsbedingungen dunkelgefärbte Produkte.

Bei der Anwendung von Hydroxypolycarbonsäuren nach US-PS 4 465 828 werden ebenfalls mit Stärke unter verschärften Reaktionsbedingungen dunkle Produkte erhalten.

In EP-PS 132 046 wird ein Verfahren zur Herstellung von Alkylglycosiden beschrieben, bei dem die Farbqualität dadurch verbessert wird, daß der Reaktionsansatz nach der Reaktion mit einem Alkalialkoholat neutralisiert wird. Bei diesem Verfahren werden die optisch detektierbaren Nebenprodukte um etwa die Hälfte vermindert.

Eine Farbregulierung von Alkylglycosiden wird in EP-PS 165 721 beschrieben. Dabei wird mit Wasserstoffperoxid gebleicht und anschließend die Farbe mit einer $SO_2$-freisetzenden Verbindung stabilisiert. Dieses Verfahren liefert jedoch Produkte, durch die bei einer Umglycosidierung dunkel gefärbte Alkylglycoside erhalten werden.

Aufgabe der vorliegenden Erfindung ist es, ein Verfahren zur Gewinnung von farblosen oder schwach gefärbten Butylglycosiden und Butyloligoglycosiden bereitzustellen, die bei Transglycosidierungen zu Alkyloligoglycosiden mit langkettigen Alkylresten keine starken Verfärbungen verursachen.

Überraschenderweise wird die Aufgabe dadurch gelöst, daß man die nach bekannten Methoden hergestellten Butyloligoglycoside und Butylglycoside einer speziellen Extraktion unterwirft. Dabei wird das Rohprodukt zunächst durch eine Säure oder eine Base auf eine Säurezahl von 0 bis 12 mg KOH/g eingestellt. Anschließend werden die Butylglycoside und Butyloligoglycoside, ggf. nach Zusatz eines hydrophoben Lösemittels, mit Wasser aus dem Gemisch extrahiert, worauf sie aus der wäßrigen Phase nach üblichen Methoden isoliert werden.

Die Butylglycoside und Butyloligoglycoside können beispielsweise nach DE-OS 19 43 689 oder nach US-PS 3 375 243 hergestellt werden. Die hier zunächst anfallenden Lösungen von Butyloligoglucosiden in Butanol weisen eine sehr dunkle Farbe auf.

Bei der Herstellung der Butylglycoside und Butyloligoglycoside können Polysaccharide wie Stärke, Oligosaccharide oder Monosaccharide eingesetzt werden. Gut geeignete Monosaccharide sind Hexosen wie Dextrose, Glucose, Mannose oder Galaktose oder Pentosen wie Ribose, Arabinose, Xylose oder Lyxose.

Als Rohprodukt für die Extraktion können flüssige Mischungen, die überwiegend Butylglycoside und Butyloligoglycoside enthalten, oder auch Lösungen der Glycoside eingesetzt werden. Vorzugsweise verwendet man die Umsetzungsprodukte aus Saccharid und Butanol.

Die Säurezahl der rohen Mischungen oder Lösungen wird durch Zugabe von Säure oder durch Zugabe einer Base auf einen Wert von 0 bis 12 mg KOH/g eingestellt. Vorzugsweise wird eine Säurezahl von 0,01 bis 3 mg KOH/g eingestellt. Säurezahlen von 0,05 bis 1 mg KOH/g sind ganz besonders bevorzugt. Bei alkalischen Lösungen verläuft die Extraktion unbefriedigend. Bei Säurezahlen über 12 mg KOH/g besteht verstärkt die Gefahr von Glycosidspaltungen während der Extraktion. Für die Einstellung der Säurezahl wird als Säure bevorzugt Schwefelsäure oder p-Toluolsulfonsäure und als Base vorzugsweise Natronlauge verwendet. Günstig ist es, als Säure die im Herstellverfahren verwendete Säure einzusetzen.

Auf 1 Teil eingestellte Mischung oder Lösung können bis zu 2 Teile hydrophobes Lösemittel zugegeben werden. Derartige Lösemittel sind vorzugsweise aromatische Kohlenwasserstoffe wie beispielsweise Toluol, Xylol oder deren Gemische.

Die eingestellten Mischungen oder Lösungen, die ggf. hydrophobe Lösemittel enthalten, werden mit 1 bis 15 Teilen Wasser kontinuierlich oder auch diskontinuierlich extrahiert.

Wird kein oder nur wenig hydrophobes Lösemittel zugesetzt, so werden für die Extraktion große Mengen Wasser benötigt. Werden dagegen 1 bis 2 Teile hydrophobes Lösemittel zugesetzt, wird nur wenig Wasser benötigt.

Die Extraktion wird im allgemeinen bei niedrigen Temperaturen durchgeführt. Vorzugsweise liegt die Tempratur bei 10 bis 30°C.

Die Glycoside befinden sich in der wäßrigen Phase und werden mit einer Base oder basischem Ionenaustauscher neutralisiert. Eventuell noch vorhandene minimale Farbspuren können mit Hilfe von Aktivkohle entfernt werden. Diese Nachbehandlung ist jedoch meist nicht notwendig.

Die Glycoside werden nach bekannten Verfahren, wie zum Beispiel durch Eindampfen, isoliert. Die Glycoside sind farblos oder leicht gelb gefärbt und sind geruchlos.

Die Farbstoffe und andere Nebenprodukte werden in der organischen Phase aufgefunden und können durch Eindampfen isoliert werden.

Das Verfahren hat folgende Vorteile :

1. Die erhaltenen Butyloligoglycoside sind von sehr guter Farbqualität.

2. Die erhaltenen Butyloligoglycoside sind geruchlos.

3. Die erhaltenen Butyloligoglycoside sind sehr farbstabil. Bei Transglycosidierungen treten fast keine Verfärbungen auf, die Transglycosidierungsprodukte sind von hoher Reinheit.

4. Das Verfahren ist einfach und kann kontinuierlich durchgeführt werden.

Beispiele 1 bis 4, Vergleichsbeispiele A und B

In einem Autoklaven werden 1 000 Teile n-Butanol, 100 Teile native Maisstärke und 1 Teil Schwefelsäure 40 Minuten auf 165°C erhitzt. Man erhält dabei als schwarzes Produkt eine Lösung von Butyloligoglucosid (Oligomerisationsgrad ca. 1,2) in Butanol.

Für die Versuche werden jeweils 100 g der Lösung in einen Schütteltrichter gegeben und mit 100 g Toluol und der in Tabelle 1 angegebenen Menge 0,1 N Natronlauge vermischt. Anschließend wird zweimal mit 100 g Wasser ausgeschüttelt. Die Jodfarbzahlen der Extrakte sind in Tabelle 1 zusammengestellt.

Nach Neutralisation der wäßrigen Lösungen mit 0,1 N Natronlauge bzw. 0,1 N Schwefelsäure wird das Lösemittel bei 60°C im Vakuum abgedampft, wobei man Butyloligoglucosid als Sirup erhält.

## Tabelle 1

| Beispiel | 0,1 N NaOH ml | Säurezahl mg KOH/g | Jodfarbzahl Extrakt 1 | Jodfarbzahl Extrakt 2 |
|----------|---------------|--------------------|-----------------------|-----------------------|
| 1 | 5,95 | 0,33 | 1 | 1 |
| 2 | 8,925 | 0,17 | 2 | 1 |
| 3 | 10,4 | 0,08 | 4 | 1 - 2 |
| 4 | 11,9 | 0 | 7 - 10 | 2 |
| A | 13,38 | alkalisch | 15 | 2 |
| B | 14,87 | alkalisch | 15 - 20 | 2 |

Die organischen Phasen weisen eine Jodfarbzahl von 15 bis 20 auf und enthalten zusätzlich dunkelbraune Farbkörper.

**Ansprüche**

1. Verfahren zur Gewinnung von farblosen oder schwach gefärbten Butylglycosiden und/oder Butyloligoglycosiden, dadurch gekennzeichnet, daß man
   – das Rohprodukt durch eine Säure bzw. Base auf eine Säurezahl von 0 bis 12 mg KOH/g einstellt,
   – ggf. ein hydrophobes Lösemittel zusetzt,
   – die Butylglycoside und Butyloligoglycoside mit Wasser extrahiert und aus der wäßrigen Phase auf üblichem Wege isoliert.

2. Verfahren nach Anspruch 1, dadurch gekennzeichnet, daß man als Rohprodukt Umsetzungsprodukte von Sacchariden und n-Butanol einsetzt.

3. Verfahren nach den Ansprüchen 1 und 2, dadurch gekennzeichnet, daß man eine Säurezahl von 0,01 bis 3 mg KOH/g einstellt.

4. Verfahren nach Anspruch 3, dadurch gekennzeichnet, daß man eine Säurezahl von 0,05 bis 1 mg KOH/g einstellt.

5. Verfahren nach den Ansprüchen 1 bis 4, dadurch gekennzeichnet, daß man auf 1 Teil Rohprodukt bis zu 2 Teile hydrophobes Lösemittel zusetzt.

6. Verfahren nach Anspruch 5, dadurch gekennzeichnet, daß man Toluol oder Xylol verwendet.

7. Verfahren nach den Ansprüchen 1 bis 6, dadurch gekennzeichnet, daß die wäßrigen Extrakte mit einer Base oder einem basischen Ionenaustauscher neutralisiert werden.

8. Verfahren nach Anspruch 7, dadurch gekennzeichnet, daß die Extrakte nach der Neutralisation mit Aktivkohle nachbehandelt werden.

9. Verfahren nach den Ansprüchen 1 bis 8, dadurch gekennzeichnet, daß kontinuierlich extrahiert wird.

10. Verfahren nach den Ansprüchen 1 bis 9, dadurch gekennzeichnet, daß Butylglucoside und/oder Butyloligoglucoside gewonnen werden.

**Claims**

1. A process for obtaining colourless or weakly coloured butylglycosides and/or butyloligoglycosides, characterized in that
   – the crude product is adjusted by an acid or base to an acid number of 0 to 12 mg of KOH/g,
   – if desired, a hydrophobic solvent is added,
   – the butylglycosides and butyloligoglycosides are extracted using water and isolated from the aqueous phase in a customary way.

2. A process according claim 1, characterized in that reaction products of saccharides and n-butanol are employed as crude product.

3. A process according to either of claims 1 and 2, characterized in that an acid number of 0.01 to 3 mg of KOH/g is established.

4. A process according to claim 3, characterized in that an acid number of 0.05 to 1 mg of KOH/g is established.

5. A process according to any of claims 1 to 4, characterized in that up to 2 parts of hydrophobic solvent are added to 1 part of crude product.

6. A process according to claim 5, characterized in that toluene or xylene is used.

7. A process according to any of claims 1 to 6, characterized in that the aqueous extracts are neutralized using a base or a basic ion exchanger.

8. A process according to claim 7, characterized in that the extracts are subsequently treated with active carbon after neutralization.

9. A process according to any of claims 1 to 8, characterized in that the mixture is continuously extracted.

10. A process according to any of claims 1 to 9, characterized in that butylglucosides and/or butyloligoglucosides are obtained.

## Revendications

1. Procédé pour l'obtention de butyl-glycosides et/ou de butyl-oligo-glycosides incolores ou faiblement teintés, caractérisé par le fait

– que l'on ajuste le produit brut, par un acide ou par une base, à un indice d'acide de zéro à 12 mg de KOH par gramme,

– que l'on ajoute éventuellement un solubilisant hydrophobe,

– que l'on extrait à l'eau les butyl-glycosides et les butyl-oligo-glycosides et qu'on les isole par voie usuelle de la phase aqueuse.

2. Procédé selon la revendication 1, caractérisé par le fait que l'on utilise, comme produit brut, des produits résultant de la réaction de saccharides et de n-butanol.

3. Procédé selon les revendications 1 et 2, caractérisé par le fait que l'on établit un indice d'acide de 0,01 à 3 mg de KOH par gramme.

4. Procédé selon la revendication 3, caractérisé par le fait que l'on établit un indice d'acide de 0,05 à 1 mg de KOH par gramme.

5. Procédé selon les revendications 1 à 4, caractérisé par le fait que l'on ajoute, pour une partie de produit brut, jusqu'à 2 parties de solubilisant hydrophobe.

6. Procédé selon la revendication 5, caractérisé par le fait qu'on utilise le toluène ou le xylène.

7. Procédé selon les revendications 1 à 6, caractérisé par le fait que les extraits aqueux sont neutralisés avec une base ou avec un échangeur d'ions basique.

8. Procédé selon la revendication 7, caractérisé par le fait qu'après la neutralisation les extraits sont soumis à un post-traitement avec du charbon actif.

9. Procédé selon les revendications 1 à 8, caractérisé par le fait que l'on extrait en continu.

10. Procédé selon les revendications 1 à 9, caractérisé par le fait que l'on obtient des butyl-glucosides et/ou des butyl-oligo-glucosides.